# EUROPEAN PATENT SPECIFICATION

(11) **EP 3 352 820 B1**
(45) Date of publication and mention of the grant of the patent: **09.04.2025**
(21) Application number: 16849407.8
(22) Date of filing: 20.09.2016
(51) Int. Cl.: A61M 5/20, A61M 5/145, A61M 5/168, A61M 5/315, A61M 5/155

(54) **INFUSING DEVICES, SYSTEMS, AND METHODS**
INFUSIONSVORRICHTUNGEN, SYSTEME UND VERFAHREN
DISPOSITIFS, SYSTÈMES ET PROCÉDÉS DE PERFUSION

(30) Priority: 24.09.2015 US 201562232316 P
(43) Date of publication of application: 01.08.2018
(73) Proprietor: Teleflex Life Sciences II LLC, Wilmington, Delaware 19808 (US)
(72) Inventor: SKELTON, Eugene, Dublin 18 (IE); BENSON, Ronald, Dublin 24 (IE); CORNER, Darren, High Peak Derbyshire SK23 7AT (GB); ALLEN, Chris, Bellevue - Islandbridge Dublin 8 (IE); KILCOIN, Christopher Brian, South Lake Tahoe, California 96158-1225 (US)
(74) Representative: Atout PI Laplace
(86) International application number: PCT/US2016/052585
(87) International publication number: WO 2017/053261

(56) References cited:
- EP-B1- 0 144 625
- WO-A1-88/10129
- DE-A1- 2 112 654
- DE-U1- 202014 001 525
- GB-A- 1 026 593
- US-A- 2 221 739
- US-A- 3 279 653
- US-A- 3 474 787
- US-A- 3 605 745
- US-A- 4 608 042
- US-A1- 2007 100 281
- US-A1- 2015 182 702
- US-B1- 6 730 060

## Description

### TECHNICAL FIELD

The present application is generally related to the technical field of intraosseous infusion, and more particularly to a device for dispensing a volume (e.g., a volume of medication or other liquid) during intraosseous infusion.

### BACKGROUND

Intraosseous (IO) infusion of fluids may in some instances be painful for patients. A small volume of lidocaine may therefore be injected after insertion of an IO needle but before infusion of other fluids or medication. Such lidocaine infusion is typically performed manually by a medical professional using a syringe, which may result in uneven and/or painful infusion. A prior art injector is disclosed in document US 6 730 060 B1.

### SUMMARY

The present invention is defined in the independent claim. Preferable embodiments are further laid out in the dependent claims. The present disclosure includes systems, methods, and apparatuses for dispensing a volume (e.g., a volume of liquid or medication) during intraosseous infusion. The various systems, methods, and apparatuses disclosed herein include various structures to control the rate at which a volume is dispensed during intraosseous infusion. Such structures are configured to provide an even dispense rate which may minimize or reduce pain caused to the patient receiving treatment. Additionally, such structures may simplify the task of performing intraosseous infusion, thereby making it easier for the medical professional to perform such a procedure.

In various embodiments, an apparatus includes a base configured to be coupled to a syringe having a body defining a reservoir and plunger slidably coupled to the body. Additionally, the apparatus includes an arm coupled to the base. A portion of the arm may be movable between an extended position and a contracted position in which the portion of the arm is closer to the base than in the extended position. The apparatus may include a resilient member coupled to base and to the arm. The resilient member may be configured to bias the portion of the arm toward the contracted position relative to the base with a force sufficient to depress the plunger of the syringe, thereby causing a volume to be dispensed from the syringe. In some embodiments, the apparatus may include a damper configured to resist movement of the portion of the arm from the extended position toward the contracted position to control a rate at which the resilient member can move the portion of the arm from the extended position toward the contracted position, thereby limiting the rate at which the volume is dispensed from the syringe. In other embodiments, the apparatus includes flow control valve coupled downstream of an output of the syringe, where the flow control valve limits the rate at which the volume is dispensed from the syringe.

An infuser or dispensing device according to the disclosed embodiments may provide a tool that may be used to dispense a volume of fluid (e.g., lidocaine or other medications) during intraosseous infusion (or another procedure) at a rate that is easily and automatically controlled (e.g., using the resilient member in connection with a damper or flow control valve), and that may be stopped or paused periodically if desired without significant disruption or hassle. Additionally, because dispensing devices configured according to the disclosed embodiments automatically control or limit the rate at which the volume is dispensed, the intraosseous infusion process may be performed more easily and with less discomfort to the patient.

The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified (and includes what is specified; e.g., substantially 90 degrees includes 90 degrees and substantially parallel includes parallel), as understood by a person of ordinary skill in the art. In any disclosed embodiment, the terms "substantially," "approximately," and "about" may be substituted with "within [a percentage] of" what is specified, where the percentage includes .1, 1, 5, and 10 percent.

Further, a device or system that is configured in a certain way is configured in at least that way, but it can also be configured in other ways than those specifically described.

The terms "comprise" (and any form of comprise, such as "comprises" and "comprising"), "have" (and any form of have, such as "has" and "having"), "include" (and any form of include, such as "includes" and "including"), and "contain" (and any form of contain, such as "contains" and "containing") are open-ended linking verbs. As a result, an apparatus that "comprises," "has," "includes," or "contains" one or more elements possesses those one or more elements, but is not limited to possessing only those elements. Likewise, a method that "comprises," "has," "includes," or "contains" one or more steps possesses those one or more steps, but is not limited to possessing only those one or more steps.

Any embodiment of any of the apparatuses, systems, and methods can consist of or consist essentially of - rather than comprise/include/contain/have - any of the described steps, elements, and/or features. Thus, in any of the claims, the term "consisting of" or "consisting essentially of" can be substituted for any of the open-ended linking verbs recited above, in order to change the scope of a given claim from what it would otherwise be using the open-ended linking verb.

The feature or features of one embodiment may be applied to other embodiments, even though not described or illustrated, unless expressly prohibited by this disclosure or the nature of the embodiments.

Some details associated with the embodiments described above and others are described below.

### BRIEF DESCRIPTION OF THE DRAWINGS

The following drawings illustrate by way of example and not limitation. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. The figures are drawn to scale (unless otherwise noted), meaning the sizes of the depicted elements are accurate relative to each other for at least the embodiment depicted in the figures.
FIG. 1A is a side view of a first embodiment of an apparatus for dispensing a volume during intraosseous infusion;
FIG. 1B is a side view of components of a deceleration chamber of the first embodiment;
FIG. 1C is a top view of the first embodiment;
FIG. 1D is an axial view of the first embodiment;
FIG. 1E is a first perspective view of the first embodiment;
FIG. 1F is a second perspective view of the first embodiment;
FIG. 1G is an illustration of an exploded view of the first embodiment;
FIG. 2A is a side view of a second embodiment of an apparatus for dispensing a volume during intraosseous infusion;
FIG. 2B is a perspective view of internal components of the second embodiment;
FIG. 2C is an axial view of the second embodiment;
FIG. 2D a first perspective view of the second embodiment;
FIG. 2E is a second perspective view of the second embodiment;
FIG. 2F is a cross section view of the second embodiment;
FIG. 2G is another side view of the second embodiment;
FIG. 2H is another perspective view of the second embodiment;
FIG. 3A is a side view of a third embodiment of an apparatus for dispensing a volume during intraosseous infusion;
FIG. 3B is a top view of the third embodiment;
FIG. 3C is an axial view of the third embodiment;
FIG. 3D is a perspective view of the third embodiment;
FIG. 3E a second perspective view of the third embodiment;
FIG. 3F is a cross sectional view of a third embodiment;
FIG. 4A is a side view of a fourth embodiment of an apparatus for dispensing a volume during intraosseous infusion;
FIG. 4B is a top view of the fourth embodiment;
FIG. 4C is an axial view of the fourth embodiment;
FIG. 5A is a side view of a fifth embodiment of an apparatus for dispensing a volume during intraosseous infusion; and
FIG. 5B is a top view of the fifth embodiment.

### DETAILED DESCRIPTION

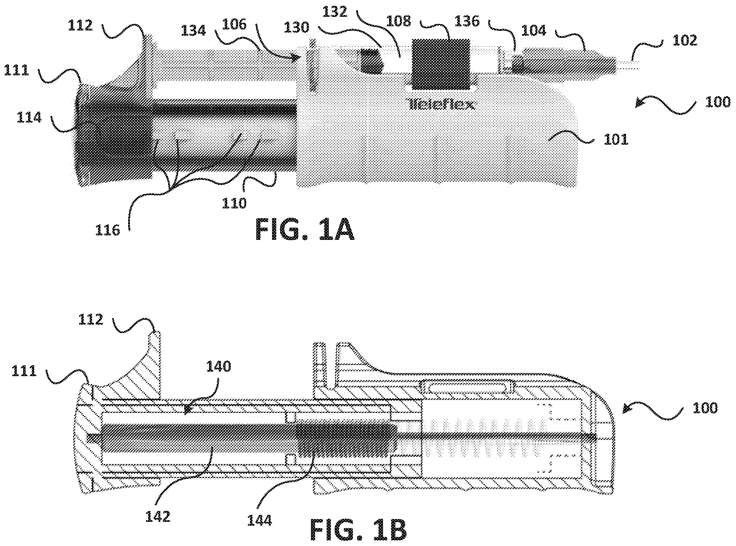

Referring to FIG. 1A, a side view of a first embodiment of an apparatus for dispensing a volume during intraosseous infusion is shown as dispensing device 100. As shown in FIG. 1A, dispensing device 100 includes a base 101 and an arm 110 coupled to base 101. Base 101 may be configured to be coupled to a syringe. The syringe may have a body 130 defining a reservoir 132 and a plunger 134 slidably coupled to body 130. Additionally, the syringe may include a tip portion 136. Tip portion 136 may be configured to be coupled to a tube 102 via a coupler 104. As plunger 134 is pulled back, a volume (e.g., a volume of fluid, such as a medication or other liquid) may be drawn through tip portion 136 of the syringe and into reservoir 132, and as plunger 134 is depressed, a portion of the volume retained in reservoir 132 may be dispensed from the reservoir. It is noted that reservoir 132 may be at least partially filled with a volume prior to or after the syringe is coupled to base 101.

In an embodiment, base 101 may define a channel 106 configured to prevent movement of the syringe along a length of base 101 as plunger 134 is depressed. In an additional or alternative embodiment, base 101 may include a strap 108 that may be configured to secure the syringe to base 101. In yet another additional or alternative embodiment, base 101 may include both channel 106 and strap 108. In an embodiment, base 101 may include one or more incremental stops 116. Each of the one or more incremental stops 116 may be configured to limit a distance by which the portion of arm 110 can compress plunger 134.

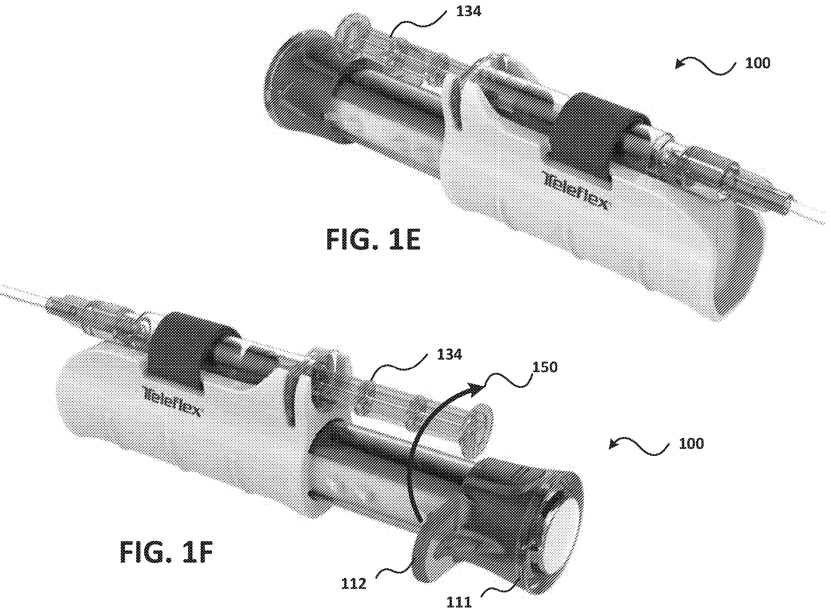

A portion of arm 110 may be movable between an extended position and a contracted position in which the portion of arm 110 is closer to the base than in the extended position, as described in more detail below. In an embodiment, arm 110 may include a tab 112. Tab 112 may be configured to contact the plunger and depress the plunger as the portion of arm 110 is moved towards the contracted position. In an embodiment, arm 112 may include a rotatable portion 111, and tab 112 may be located on rotatable portion 111. This enables tab 112 to be rotated (as indicated by the arrow 150 of FIG. 1F) between a first position in which tab 112 is aligned with plunger 134, as shown in FIGs. 1A-1D, and a second position in which the tab is not aligned with plunger 134, as shown in FIGs. 1E and 1F. In an embodiment, arm 110 may include a lock (not shown) that may be configured to prohibit rotation of rotatable portion 111 so as to prevent the rotation of tab 112 to the second position when dispensing device 100 is dispensing a volume.

In an embodiment, arm 110 may include one or more locking tabs 114 configured to releaseably lock the portion of arm 110 at a position corresponding to one of incremental stops 116 to prohibit further movement of the portion of arm 110 towards the contracted position. In an embodiment, each of incremental stops 116 may be configured such that a distance by which the portion of arm 110 can compress plunger 134 corresponds to dispensing of a predetermined volume from reservoir 132. For example, incremental stops 116 may be spaced such that each incremental stop 116 represents an incremental volume (e.g., a 1 milliliter (mL) volume) being dispensed from the syringe (e.g., the distance by which arm 110 can compress plunger 134 between adjacent incremental stops results in a 1 milliliter volume being dispensed from the syringe), such that upon releasing the portion of arm 110 using the one or more locking tabs 114, the portion of arm 110 will move towards the contracted position and become locked at the next adjacent incremental stop 116, resulting in 1 mL volume being dispensed from the syringe. In an additional or alternative embodiment, the distance between adjacent incremental stops 116 may not be related to a defined volume being dispensed from the syringe, and instead may simply provide a mechanism for stopping or pausing the dispensing of the volume.

Referring briefly to FIG. 1B, a side view illustrating components of a deceleration chamber of the first embodiment the apparatus for dispensing a volume during intraosseous infusion is shown as a deceleration chamber 140. In some embodiments, deceleration chamber 140 may be housed within the base 101, as shown in FIG. 1B. In the embodiment shown, deceleration chamber 140 includes a resilient member 144 and a damper 142. Resilient member 144 may be coupled to base 101 and to arm 110, and may be configured to bias the portion of arm 110 toward the contracted position relative to base 101 with a force sufficient to depress plunger 134 of the syringe that is coupled to base 101. Damper 142 may be configured to resist movement of the portion of arm 110 from the extended position toward the contracted position to control a rate at which resilient member 144 can move the portion of arm 110 from the extended position toward the contracted position.

In an embodiment, resilient member 144 may include a spring configured to exert a first force on arm 110, and damper 142 may include a piston configured to exert a second force on arm 110. The second force may oppose the first force to resist movement of the portion of arm 110 from the extended position toward the contracted position. The difference between the first force and the second force may be configured to limit a rate at which the portion of arm 110 depresses plunger 134.

During operation, a user (e.g., a medical professional) of the dispensing device 100 may couple base 101 to a syringe, and may extend the portion of arm 110 to the extended position, as shown in FIG. 1A. In an embodiment, the user may lock the portion of arm 110 in the extended position using incremental stops 116. A compressive force may be applied to the spring (e.g., the resilient member 144 of FIG. 1B) when the portion of arm 110 is in the extended position. The user may release the compressive force (e.g., using locking tabs 114) to allow the spring to expand and move the portion of arm 110 with a force sufficient to depress plunger 134. As the spring expands, plunger 134 is depressed by the portion of arm 110 and a volume is dispensed into tube 102. If the user desires to continuously dispense the volume held in reservoir 132, the user may maintain locking tabs 114 in an orientation such that locking tabs 114 do not engage and lock onto a next adjacent one of the one or more incremental stops 116. In such a scenario, the rate at which the volume is continuously dispensed is controlled by resilient member 144 and damper 142 (e.g., the spring and the piston). If the user desires to pause or stop dispensing the volume, the user may release locking tabs 114, which will cause the portion of arm 110 to stop moving towards the contracted position when locking tabs 114 lock onto the next adjacent one of incremental stops 116.

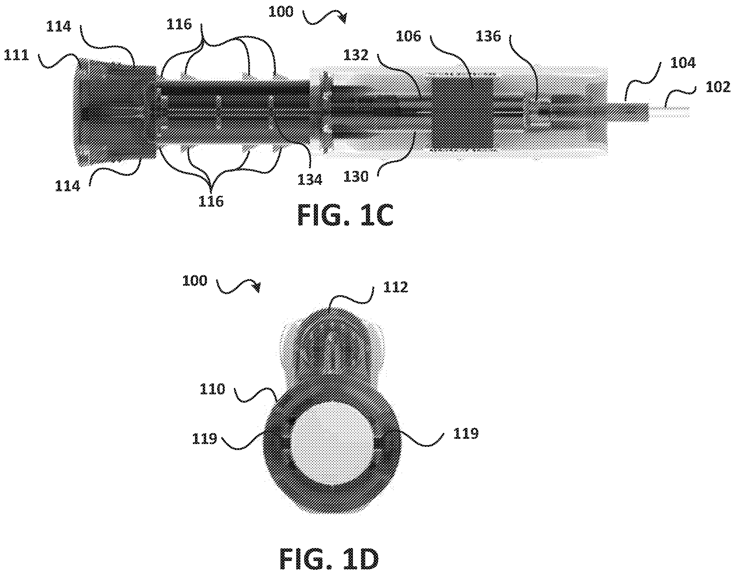

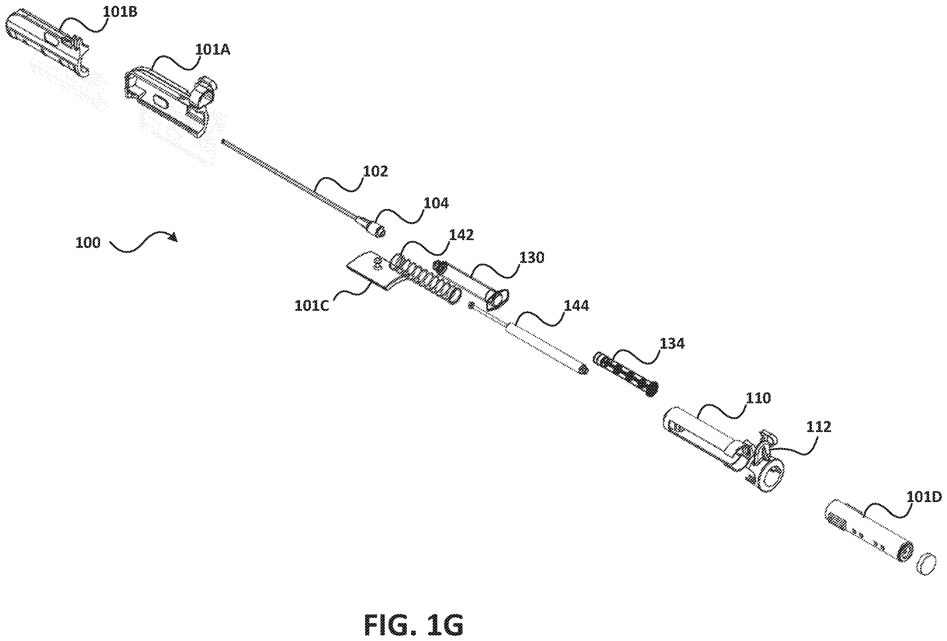

Referring to FIG. 1G, an illustration of an exploded view of the first embodiment of an apparatus for dispensing a volume during intraosseous infusion. As illustrated in FIG. 1G, base 101 may be formed of multiple component parts 101A-101D. Additionally, as shown in FIG. 1G, in an embodiment, arm 110 may be configured as a sleeve that slides over a component 101D of base 101. Referring briefly to FIG. 1D, it can be seen that, in an embodiment, arm 110 may include apertures 119 that may be configured allow incremental stops 116 located on the component 101D to pass through the end of arm 110 as the portion of arm 110 moves towards the contracted position.

FIGs. 1C and 1E provide additional views of the first embodiment of dispensing device 100 described above. While incremental stops 116 are shown on two sides of the base 101, in other embodiments, one or more incremental stops 116 may be located on a single side of the base 101, or on more than two sides of the base 101. Additionally, arm 110 may include one or more locking tabs 114 depending on number of locations or sides of base 101 having incremental stops 116.

From the foregoing description, it has been shown that dispensing device 100 provides a tool that may be used to dispense a volume of fluid (e.g., lidocaine or other medications) during intraosseous infusion at a rate that is easily controllable, and that may be stopped or paused periodically if desired without significant disruption or hassle. Additionally, because dispensing device 100 automatically controls or limits the rate at which the fluid or volume is dispensed using the resilient member and the damper, the intraosseous infusion process may be performed more easily and with less discomfort to the patient. Further, it is noted that although described as a tool for improving intraosseous infusion, one of ordinary skill in the art would readily recognize that dispensing device 100 may be readily adapted and used for purposes other than intraosseous infusion.

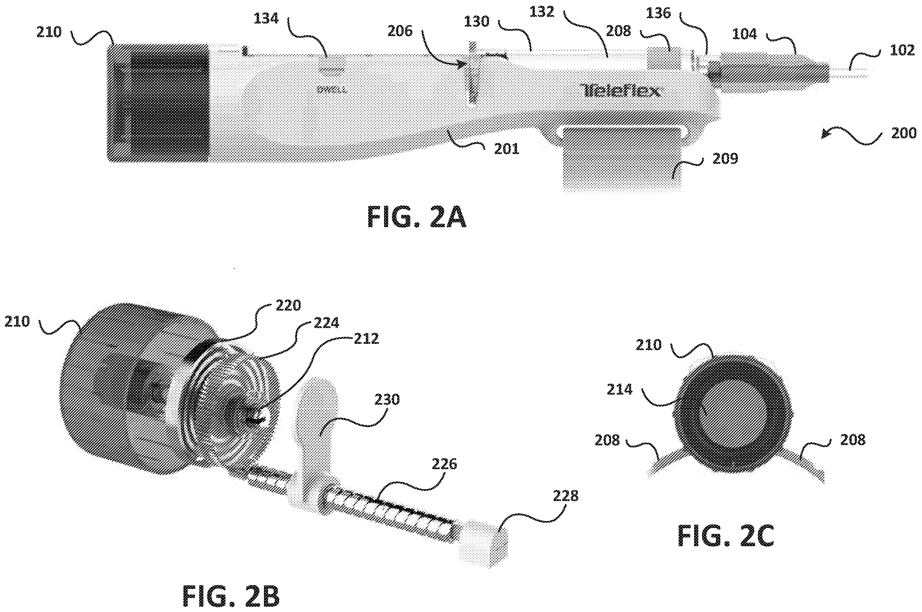

Referring to FIG. 2A, a side view of a second embodiment of an apparatus for dispensing a volume during intraosseous infusion is shown as a dispensing device 200. As shown in FIG. 2A, dispensing device 200 includes a base 201 and a primer 210 coupled to base 201. Base 201 may be configured to be coupled to a syringe (as described above).

In an embodiment, the base 201 may define a channel 206 configured to prevent movement of the syringe along a length of base 201 as plunger 134 is depressed. In some embodiments, base 201 may include a strap 208 that may be configured to secure the syringe to base 201. In some embodiments, base 201 may include both channel 206 and strap 208. In some embodiments, base 201 may be configured to be coupled to a second strap 209 configured to secure base 201 to a patient's arm or leg, or to another tool or piece of equipment (not shown).

Dispensing device 200 may include a resilient member and a damper that may be housed within base 201. For example, and as shown in FIG. 2B, the additional components of dispensing device 200 may include a clutch 212, a spiral torsion spring 220, one or more gears 224, a rotary drive 226, a damper 228, and an arm 230. Clutch 212 may, for example, comprise a slip clutch.

A portion of arm 230 may be movable between an extended position (left side or closer to spiral torsion spring 220) and a contracted position (right side or closer to damper 228) in which the portion of the arm is closer to the base than in the extended position. The portion of arm 230 may be configured to contact the plunger to depress the plunger. The resilient member may be coupled to base 201 and to the portion of arm 230, and may be configured to bias the portion of arm 230 toward the contracted position relative to base 201 with a force sufficient to depress the plunger 134 of the syringe that is coupled to base 201. Damper 228 may be configured to resist movement of the portion of arm 230 from the extended position toward the contracted position to control a rate at which the resilient member can move the portion of arm 230 from the extended position toward the contracted position.

In an embodiment, the resilient member may include spiral torsion spring 220 coupled to rotary drive 226, and may be configured to exert a first force, and damper 228 may include a rotary damper configured to exert a second force. The second force may oppose the first force to resist movement of the portion of arm 230 from the extended position toward the contracted position. The difference between the first force and the second force may be configured to limit a rate at which the portion of arm 230 depresses plunger 134.

Clutch 212 may be configured to selectively engage spiral torsion spring 220. In an embodiment, spiral torsion spring 220 may be primed by rotating primer 210. For example, when clutch 212 is engaging spiral torsion spring 220, primer 210 may be rotated to prime (e.g., wind) spiral torsion spring 220. When clutch 212 is disengaged from the spiral torsion spring 220, spiral torsion spring 220 may begin unwinding, which rotates rotary drive 226 in a first direction and moves the portion of arm 230 along a length of rotary drive 226 with a force sufficient to depress plunger 134. To illustrate, as spiral torsion spring 220 unwinds, gear 224 may begin to rotate which causes rotary drive 226 to rotate in the first direction. As the rotary drive 226 rotates in the first direction, the portion of the arm 230 may move along the length of the rotary drive 226 (along helical threads of the rotary drive) towards the contracted position (e.g., to the right in FIG. 2B) with a force sufficient to depress plunger 134. In some embodiments, the priming of spiral torsion spring 220 while clutch 212 is engaged may cause rotary drive 226 to rotate in a second direction, and, as rotary drive 226 rotates in the second direction, the portion of arm 230 may be moved along the length of rotary drive 226 towards the extended position (e.g., towards primer 210), thereby preparing dispensing device 200 for dispensing a volume from the syringe.

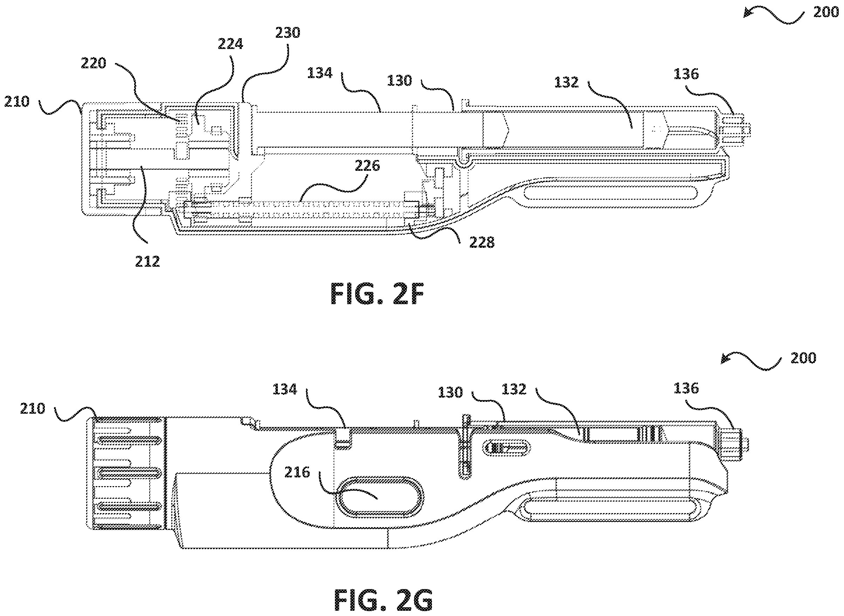

In an embodiment, dispensing device 200 may include a control (e.g., a button, etc.) that, when activated, may cause clutch 212 to engage or disengage spiral torsion spring 220. For example, as shown in FIG. 2C, primer 210 may include a control 214 (e.g., a button, etc.) that, when depressed, may selectively cause clutch 212 to engage or disengage spiral torsion spring 220. For example, a first press of control 214 may cause clutch 212 to engage spiral torsion spring 220 for priming using primer 210, and a second press of control 214 may disengage clutch 212 from spiral torsion spring 220 to allow spiral torsion spring 220 to unwind. While control 214 is shown on an end of the primer 210, other embodiments may include a control for selectively causing clutch 212 to engage and/or disengage the resilient member located in other locations of dispensing device 200. For example, FIG. 2G illustrates a side view of a variation of dispensing device 200 having a control 214 configured to cause clutch 212 to engage and/or disengage the resilient member. Additionally, in an embodiment, clutch 212 may be located at a different location than the location illustrated in FIG. 2C. For example, in an additional or alternative embodiment, the clutch may be configured to engage rotary drive 226 to prime spiral torsion spring 220 (e.g,. by rotating rotary drive 226 in the second direction). While engaged, the clutch may prevent rotation of rotary drive 226 in the first direction, which may prohibit spiral torsion spring 220 from unwinding until clutch 212 is disengaged.

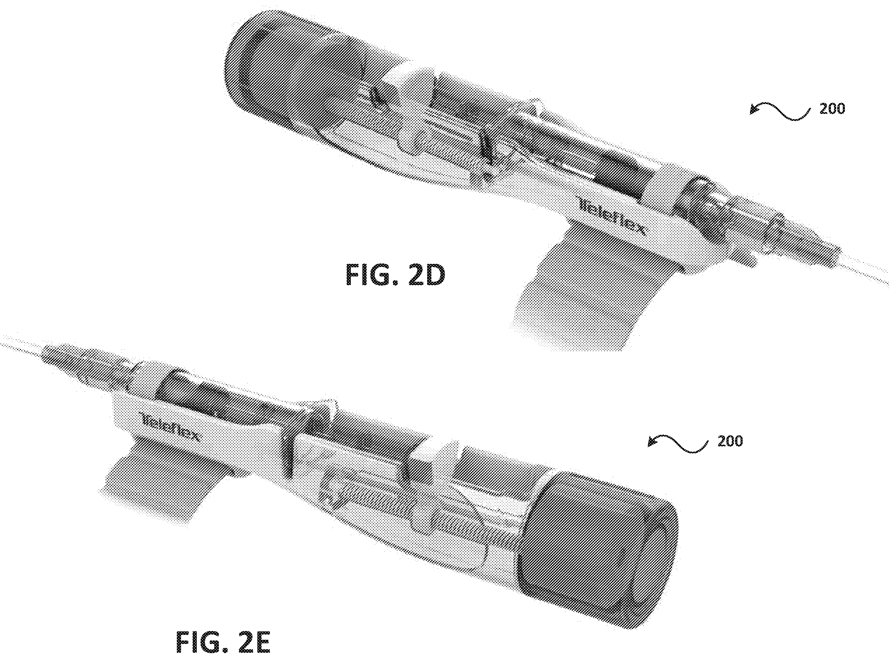

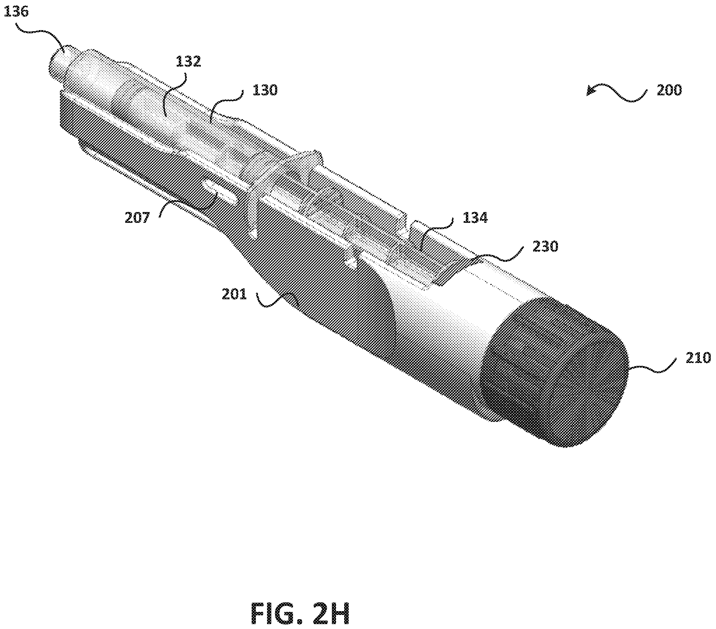

FIGs. 2D and 2E illustrate perspective views of the dispensing device 200. FIG. 2F is a cross sectional view of the second embodiment of an apparatus for dispensing a volume during intraosseous infusion. In FIG. 2F it can be seen that the resilient member (e.g., spiral torsion spring 220 and rotary drive 226), as well as gear(s) 224 and damper 228 may be housed within base 201. Referring briefly to FIG. 2H, another perspective view of dispensing device 200 is shown. As shown in FIG. 2H, base 201 of dispensing device 200 may define a channel along its length, and the syringe, when coupled to based 201, may rest in the channel. The longitudinal channel may be sized and dimensioned to allow arm 230 to move between the extended position (shown in FIG. 2H) and the contracted position within the longitudinal channel. In other embodiments, base 201 may further define a second channel (e.g., channel 206 of FIG. 2A) that is configured to prohibit movement of the syringe as the plunger 134 is depressed. In another additional or alternative embodiment, the base 201 may define apertures 207 configured to receive the strap 208 of FIG. 2A. In still another additional or alternative embodiment, the base 201 may define the longitudinal channel along the length of the base, the channel 206, the aperture(s) 207, or a combination thereof. Additionally, the embodiments illustrated in FIGs. 1A-1G may, in some variations, have a base that defines a longitudinal channel similar to the longitudinal channel illustrated in FIG. 2H.

During operation, a user (e.g., a medical professional) of dispensing device 200 may be couple base 201 to a syringe, and may prime the resilient member, as described above. When the user desires to initiate dispensing of a volume from the syringe by activating control 214 (or control 216), which may disengage clutch 212 to enable the resilient member to bias the portion of arm 230 towards the contracted position with a force sufficient to dispense the volume from the syringe.

From the foregoing description, it has been shown that dispensing device 200 provides a tool that may be used to dispense a volume of fluid (e.g., lidocaine or other medications) during intraosseous infusion at a rate that is easily controllable, and that may be stopped or paused periodically if desired without significant disruption or hassle, using the clutch for example. Additionally, because dispensing device 200 automatically controls or limits the rate at which the fluid or volume is dispensed using the resilient member and the damper, the intraosseous infusion process may be performed more easily and with less discomfort to the patient. Further, it is noted that although described as a tool for improving intraosseous infusion, one of ordinary skill in the art would readily recognize that dispensing device 200 may be readily adapted and used for purposes other than intraosseous infusion.

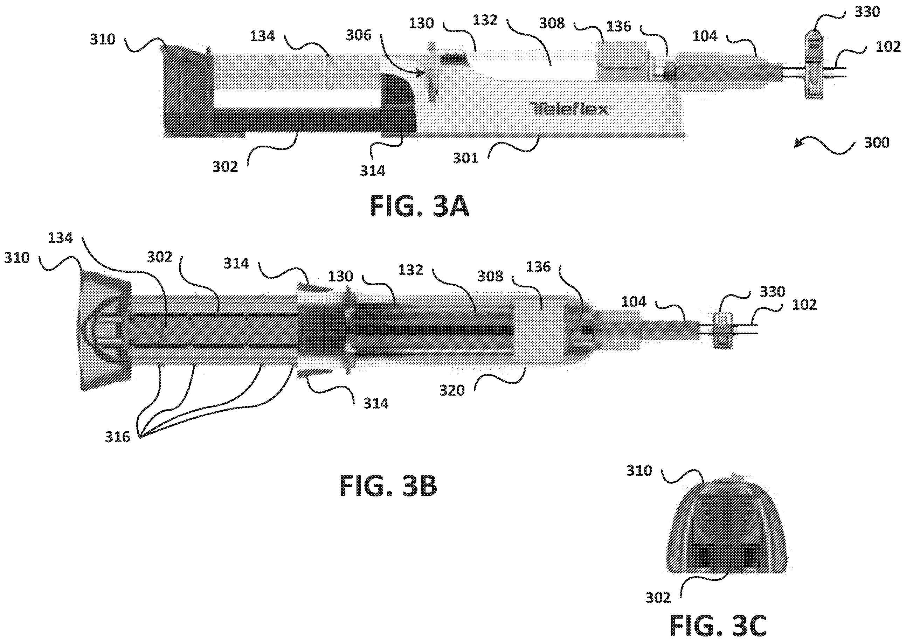

Referring to FIG. 3A, a side view of a third embodiment of an apparatus for dispensing a volume during intraosseous infusion is shown as a dispensing device 300. As shown in FIG. 3A, dispensing device 300 includes a base 301 and an arm 310 coupled to base 301. Base 301 may be configured to be coupled to a syringe (as described above).

In an embodiment, base 301 may define a channel 306 configured to prevent movement of the syringe along a length of base 301 as the plunger 134 is depressed. In some embodiments, base 301 may include a strap 308 that may be configured to secure the syringe to the base 301. In some embodiments, base 301 may include both channel 306 and strap 308.

A portion of arm 310 may be movable between an extended position and a contracted position in which the portion of arm 310 is closer to base 301 than in the extended position. For example, FIGs. 3A and 3B illustrate the portion of arm 310 in the extended position, and FIGs. 2D-3F illustrate the portion of arm 310 in the contracted position. Dispensing device 300 may include a resilient member 302 that may be coupled to base 301 and arm 310, and may be configured to bias the portion of arm 310 toward the contracted position relative to base 301 with a force sufficient to depress plunger 134 of the syringe that is coupled to base 301. FIGs. 2B and 2C illustrate additional views of resilient member 302 that is coupled to base 301 and arm 310. In some embodiments, resilient member 302 may include an elastic band. As the portion of arm 310 is moved towards the extended position, the elastic band may stretch, and, upon initiating dispensing of the volume from the syringe (e.g., by unlocking the portion of arm 310 using locking tabs 314 or otherwise), the elastic band may move the portion of arm 310 towards the contracted position with a force sufficient to depress plunger 134 of the syringe.

In the embodiment shown, arm 310 may include one or more incremental stops 316, and base 301 may include one or more locking tabs 314. Locking tabs 314 may be configured to releaseably lock the portion of arm 310 at a position corresponding to one of the incremental stops 316 to prohibit further movement of the portion of arm 310 towards the contracted position. In some embodiments, ach of the incremental stops 316 may be configured such that a distance by which the portion of arm 310 can compress plunger 134 corresponds to dispensing of a predetermined volume from reservoir 132. For example, incremental stops 316 may be spaced such that each incremental stop 316 represents an incremental volume (e.g., a 1 milliliter (mL) volume) being dispensed from the syringe (e.g., the distance by which arm 310 can compress plunger 134 between adjacent incremental stops results in a 1 milliliter volume being dispensed from the syringe), such that upon releasing the portion of arm 310 using the one or more locking tabs 314, the portion of arm 310 will move towards the contracted position and become locked at the next adjacent incremental stop 316, resulting in 1 mL volume being dispensed from the syringe. In an additional or alternative embodiment, the distance between adjacent incremental stops 316 may not be related to a defined volume being dispensed from the syringe, and instead may simply provide a mechanism for stopping or pausing the dispensing of the volume.

In an embodiment, dispensing device 300 may include a flow control valve 330 configured to be coupled downstream of an outlet (e.g., tip portion 136) of the syringe and to control a rate at which a volume is dispensed from the syringe. For example, as shown in FIG. 3A, flow control valve 330 may be coupled to tip portion 136 of the syringe via coupler 104, and an output of flow control valve 330 may be coupled to tube 102. Flow control valve 330 may be configured to limit the flow of the volume dispensed from the syringe such that the volume is dispensed at a desired rate. It is noted that in other embodiments, a damper may be used to control the rate at which the volume is dispensed from the syringe. For example, in some embodiments, dispensing device 300 may include a damper (e.g., damper 142 of FIG. 1B) coupled to arm 310 and base 301. The damper may be configured to resist movement of the portion of arm 310 from the extended position toward the contracted position to control a rate at which resilient member 302 can move the portion of the arm from the extended position toward the retracted position.

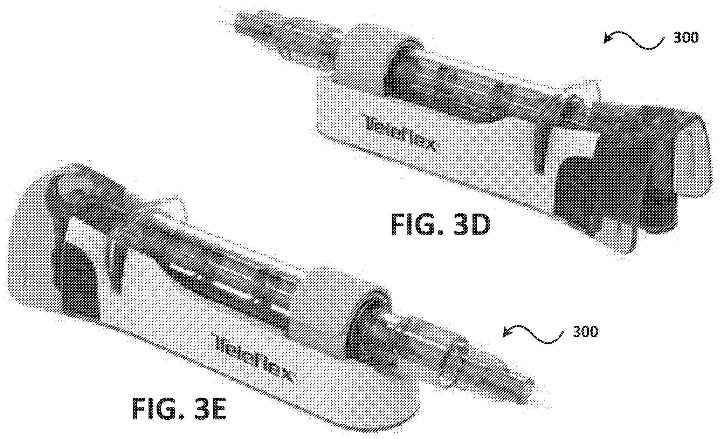

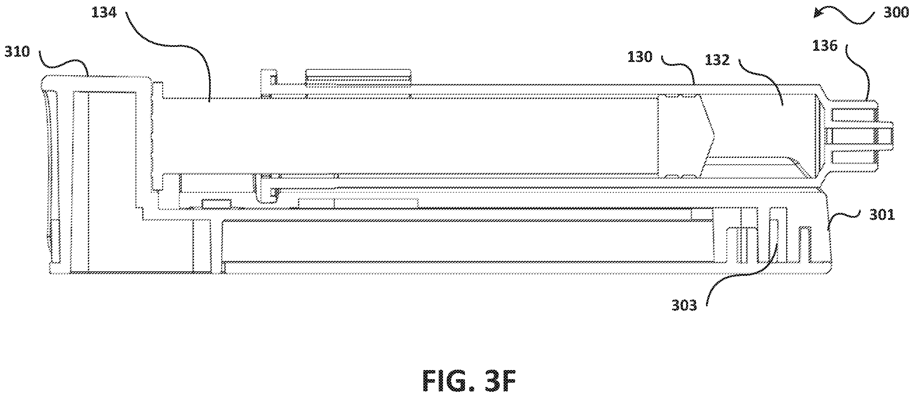

FIGs. 3D and 3E illustrate perspective view of dispensing device 300 in the contracted position. Referring briefly to FIG. 3F, a cross sectional view of dispensing device 300 is shown. As shown in FIG. 3F, in an embodiment, base 301 may define one or more posts 303 that may be used to couple resilient member 302 to base 301. Additionally, arm 310 may define one or more posts (not labeled in FIG. 3F) that may be used to couple resilient member 302 to arm 310.

During operation, a user (e.g., a medical professional) of dispensing device 300 may couple base 301 to a syringe, and may extend the portion of arm 310 to the extended position, as shown in FIG. 3A. In an embodiment, the user may lock the portion of arm 310 in the extended position using incremental stops 316. A force may be applied to resilient member 302 when the portion of the arm 310 is in the extended position. The user may release the force (e.g., using locking tabs 314) to allow resilient member 302 to contract and move the portion of arm 310 with a force sufficient to depress plunger 134. As resilient member 302, plunger 134 is depressed by the portion of arm 310 and a volume may be dispensed into tube 102. In an embodiment, the rate at which the volume is dispensed into tube 102 may be limited by flow control valve 330. If the user desires to continuously dispense the volume held in the reservoir, the user may maintain locking tabs 314 in an orientation such that locking tabs 314 do not engage and lock onto a next adjacent one of incremental stops 316. In such a scenario, the rate at which the volume is continuously dispensed is controlled by resilient member 302 and a damper (e.g., flow control valve 330 or another component configured to resist movement of the portion of arm 310 from the extended position to the contracted position). If the user desires to pause or stop the dispensing of the volume, the user may release locking tabs 314, which will cause the portion of arm 310 to stop moving towards the contracted position when locking tabs 314 lock onto the next adjacent one of incremental stops 316.

From the foregoing description, it has been shown that dispensing device 300 provides a tool that may be used to dispense a volume of fluid (e.g., lidocaine or other medications) during intraosseous infusion at a rate that is easily controllable, and that may be stopped or paused periodically if desired without significant disruption or hassle. Additionally, because dispensing device 300 automatically controls or limits the rate at which the fluid or volume is dispensed using resilient member 302 and the damper, the intraosseous infusion process may be performed more easily and with less discomfort to the patient. Further, it is noted that although described as a tool for improving intraosseous infusion, one of ordinary skill in the art would readily recognize that dispensing device 300 may be readily adapted and used for purposes other than intraosseous infusion.

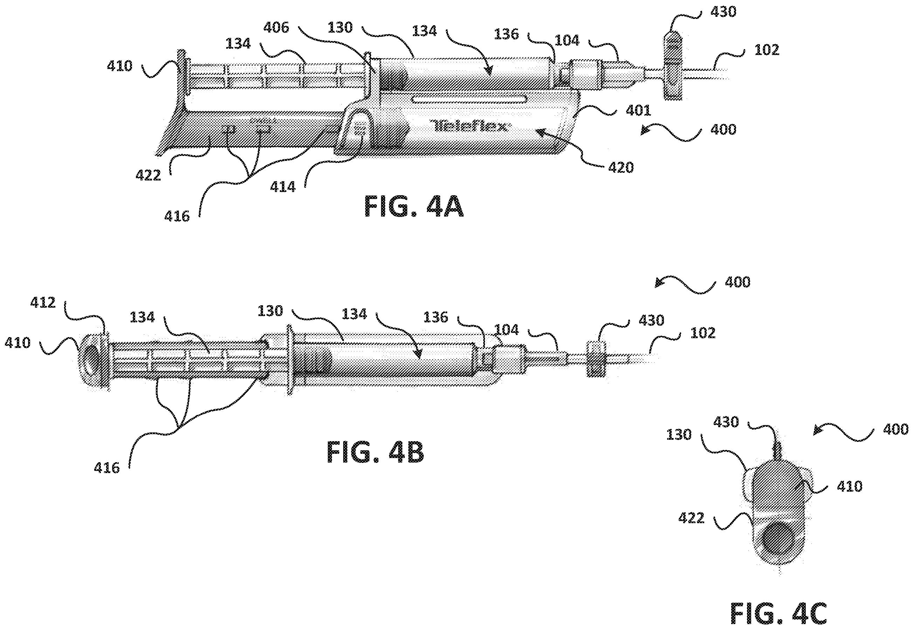

Referring to FIG. 4A, a side view of a fourth embodiment of an apparatus for dispensing a volume during intraosseous infusion is shown as a dispensing device 400. As shown in FIG. 4A, dispensing device 400 includes a base 401 configured to be coupled to a syringe (as described above). In this embodiment, base 401 defines a vacuum chamber 420. Additionally, dispensing device 400 includes an arm 410 coupled to base 401 and a piston 422. A portion of arm 410 may be movable between an extended position and a contracted position in which the portion of arm 410 is closer to base 401 than in the extended position. Piston 422 may be at least partially disposed in vacuum chamber 420 and coupled in fixed relation to the portion of arm 410. Dispensing device 400 may be configured such that as the portion of arm 410 is moved from the contracted position to the extended position, pressure within vacuum chamber 420 decreases below an atmospheric pressure to a point at which piston 422 and portion of arm 410 are biased toward the contracted position with a force sufficient to depress plunger 134 of the syringe. In an embodiment, dispensing device 400 may include a flow control valve 430 that is configured to be coupled downstream of an outlet (e.g., the portion 136) of the syringe and to control a rate at which a volume (e.g., a volume of fluid) is dispensed from the syringe.

In an embodiment, piston 422 may include one or more incremental stops 416, and base 401 may include one or more locking tabs 414. Locking tabs 414 may be configured to releaseably lock the portion of arm 410 at a position corresponding to one of incremental stops 416 to prohibit further movement of the portion of arm 410 towards the contracted position. In an embodiment, each incremental stop 416 may be configured such that a distance by which the portion of arm 410 can compress plunger 134 corresponds to dispensing of a predetermined volume from reservoir 132. For example, incremental stops 416 may be spaced such that each incremental stop 416 represents an incremental volume (e.g., a 1 milliliter (mL) volume) being dispensed from the syringe (e.g., the distance by which arm 410 can compress plunger 134 between adjacent incremental stops results in a 1 milliliter volume being dispensed from the syringe), such that upon releasing the portion of arm 410 using the one or more locking tabs 414, the portion of arm 410 will move towards the contracted position and become locked at the next adjacent incremental stop 416, resulting in 1 mL volume being dispensed from the syringe. In an additional or alternative embodiment, the distance between adjacent incremental stops 416 may not be related to a defined volume being dispensed from the syringe, and instead may simply provide a mechanism for stopping or pausing the dispensing of the volume.

FIGs. 4B and 4C illustrate additional view of dispensing device 400 of embodiments. In the embodiment shown, base 401 may define tab 406 having a channel that enables the syringe to be seated in the channel and retained in a fixed horizontal position along the length of base 401 as plunger 134 is depressed. In an additional or alternative embodiment, a strap, such as strap 108 of FIG. 1A may be used alone, or in combination with tab 406 to secure the syringe to base 401.

During operation, a user (e.g., a medical professional) of dispensing device 400 may couple base 401 to a syringe, and may extend the portion of arm 410 to the extended position, as shown in FIG. 4A. In an embodiment, the user may lock the portion of arm 410 in the extended position using incremental stops 416. As the portion of arm 410 is moved from the contracted position to the extended position, pressure within vacuum chamber 420 decreases below an atmospheric pressure to a point at which piston 422 and the portion of arm 410 are biased toward the contracted position with a force sufficient to depress plunger 134 of the syringe. The user may unlock the portion of arm 410 from the extended position using locking tabs 414 to initiate movement of the portion of arm 410 towards the contracted position with a force sufficient to depress plunger 134. As the portion of arm 410 moves towards the contracted position, plunger 134 is depressed and a volume may be dispensed into tube 102. In an embodiment, the rate at which the volume is dispensed into tube 102 may be limited by flow control valve 430. If the user desires to continuously dispense the volume held in the reservoir, the user may maintain the locking tabs 414 in an orientation such that locking tabs 414 do not engage and lock onto a next adjacent one of incremental stops 416. In such a scenario, the rate at which the volume is continuously dispensed is controlled by flow control valve 430. If the user desires to pause or stop the dispensing of the volume, the user may release locking tabs 414, which will cause the portion of arm 410 to stop moving towards the contracted position when locking tabs 414 lock onto the next adjacent one of incremental stops 416.

From the foregoing description, it has been shown that dispensing device 400 provides a tool that may be used to dispense a volume of fluid (e.g., lidocaine or other medications) during intraosseous infusion at a rate that is easily controllable, and that may be stopped or paused periodically if desired without significant disruption or hassle. Additionally, because dispensing device 400 automatically controls or limits the rate at which the fluid or volume is dispensed using flow control valve 430, the intraosseous infusion process may be performed more easily and with less discomfort to the patient. Further, it is noted that although described as a tool for improving intraosseous infusion, one of ordinary skill in the art would readily recognize that dispensing device 400 may be readily adapted and used for purposes other than intraosseous infusion.

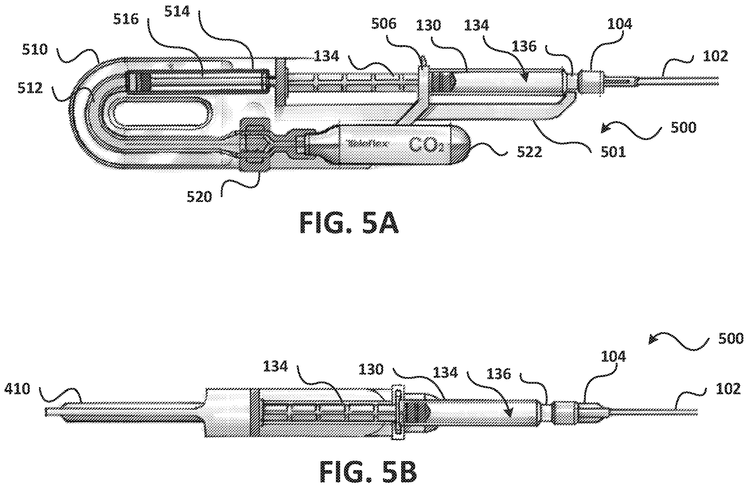

Referring to FIG. 5A, a side view of a fifth embodiment of an apparatus for dispensing a volume during intraosseous infusion is shown as a dispensing device 500. As shown in FIG. 5A, dispensing device 500 includes a base 501 configured to be coupled to a syringe (as described above). In the embodiment shown, the base may define a cylinder 514. Additionally, dispensing device 500 includes a piston 516 disposed within cylinder 514 such that piston 516 is movable between an extended position and a contracted position in which a portion of the piston is closer to base 501 than in the extended position. Base 501 may be coupled to or include a receiver 520 configured to be coupled to a source 522 of pressurized fluid. The receiver 520 may be in fluid communication with cylinder 514 to direct pressurized fluid to cylinder 514 to bias the portion of piston 516 toward the extended position relative to base 501 with a force sufficient to depress plunger 134 of the syringe. In an embodiment, source 522 of the compressed fluid includes a CO2 cylinder. FIG. 5B illustrates a top view of dispensing device 500. In the embodiment shown, base 501 may define a tab 506 having a channel that enables the syringe to be seated in the channel and retained in a fixed horizontal position along the length of base 501 as plunger 134 is depressed. In some embodiments, a strap, such as strap 108 of FIG. 1A may be used alone, or in combination with tab 506 to secure the syringe to base 501.

The above specification and examples provide a complete description of the structure and use of exemplary embodiments. Although certain embodiments have been described above with a certain degree of particularity, or with reference to one or more individual embodiments, those skilled in the art could make numerous alterations to the disclosed embodiments without departing from the scope of this invention. As such, the various illustrative embodiments of the present devices are not intended to be limited to the particular forms disclosed. Rather, they include all modifications and alternatives falling within the scope of the claims, and embodiments other than the one shown may include some or all of the features of the depicted embodiment. For example, components may be combined as a unitary structure, and/or connections may be substituted. Further, where appropriate, aspects of any of the examples described above may be combined with aspects of any of the other examples described to form further examples having comparable or different properties and addressing the same or different problems. Similarly, it will be understood that the benefits and advantages described above may relate to one embodiment or may relate to several embodiments.

The claims are not intended to include, and should not be interpreted to include, means-plus- or step-plus-function limitations, unless such a limitation is explicitly recited in a given claim using the phrase(s) "means for" or "step for," respectively.

## Claims

1. An apparatus (100) for dispensing a volume during intraosseous infusion, the apparatus comprising:
a base (101) configured to be coupled to a syringe (130) having a body defining a reservoir (132) and plunger (134) slidably coupled to the body;
an arm (110) coupled to the base (101), wherein a portion of the arm is movable between an extended position and a contracted position in which the portion of the arm is closer to the base than in the extended position;
a resilient member (144) coupled to base (101) and to the arm (110), wherein the resilient member (144) is configured to bias the portion of the arm (110) toward the contracted position relative to the base with a force sufficient to depress a plunger of a syringe coupled to the base; and
a damper (142) configured to resist movement of the portion of the arm (110) from the extended position toward the contracted position to control a rate at which the resilient member can move the portion of the arm from the extended position toward the contracted position;
where the base (101) defines a channel (106) configured to prevent movement of the syringe along a length of the base as the plunger is depressed, and the base (101) includes one or more incremental stops (116), each of the one or more incremental stops being configured to limit a distance by which the portion of the arm (110) is operable to compress the plunger (134),
where the arm (110) includes a tab (112) configured to contact the plunger (134) and depress the plunger as the portion of the arm (110) is moved towards the contracted position,
where the resilient member (144) includes a spring configured to exert a first force on the arm (110), and
where the damper (142) includes a piston configured to exert a second force on the arm (110) that opposes the first force.

2. The apparatus of claim 1, where the tab (112) is rotatable between a first position in which the tab is aligned with the plunger (134) and a second position in which the tab is not aligned with the plunger.

3. The apparatus of claim 1, where the arm includes one or more locking tabs (114) configured to releaseably lock the portion of the arm (110) at a position corresponding to one of the one or more incremental stops (116) to prohibit further movement of the portion of the arm towards the contracted position.

4. The apparatus of claim 1, where the apparatus is configured to apply a compressive force to the spring (144) when the portion of the arm (110) is in the extended position.

5. The apparatus of claim 1, where the one or more incremental stops (116) is spaced such that each incremental stop represents an incremental volume disposed from the syringe.

6. The apparatus of claim 1, where the arm (110) is configured as a a sleeve configured to slide over a component (101D) of the base.

7. The apparatus of claim 6, where the arm (110) further includes apertures configured to allow the one or more incremental stops (116) located on the component (101D) of the base to pass through an end of the arm as arm moves toward the contracted position.

8. The apparatus of claim 2, where the arm further includes a lock configured to prohibit rotation of the tab to the second position.

## Patentansprüche

1. Vorrichtung (100) zur Abgabe eines Volumens während einer intraossären Infusion, wobei die Vorrichtung umfasst:
eine Basis (101), die konfiguriert ist, um mit einer Spritze (130) gekoppelt werden zu können, die einen Körper, der ein Reservoir (132) definiert, und einen Stößel (134) aufweist, der gleitend mit dem Körper verbunden ist;
einem Arm (110), der mit der Basis (101) gekoppelt ist, wobei ein Abschnitt des Arms zwischen einer ausgefahrenen Position und einer zusammengezogenen Position beweglich ist, in der der Abschnitt des Arms näher an der Basis ist als in der ausgefahrenen Position;
ein elastisches Element (144), das mit der Basis (101) und dem Arm (110) gekoppelt ist, wobei das elastische Element (144) konfiguriert ist, um den Abschnitt des Arms (110) in Richtung der zusammengezogenen Position relativ zur Basis mit einer Kraft vorzuspannen, die ausreicht, um einen Stößel einer Spritze nieder zu drücken, die mit der Basis gekoppelt ist; und
einen Dämpfer (142), der konfiguriert ist, um einer Bewegung des Abschnitts des Arms (110) aus der ausgefahrenen Position in Richtung der zusammengezogenen Position zu widerstehen, um eine Geschwindigkeit zu steuern, mit welcher das elastische Element den Abschnitt des Arms von der ausgefahrenen Position in die zusammengezogene Position bewegen kann;
wobei die Basis (101) einen Kanal (106) definiert, der konfiguriert ist, um die Bewegung der Spritze entlang einer Länge der Basis zu verhindern, während der Stößel niedergedrückt wird, und die Basis (101) einen oder mehrere inkrementelle Anschläge (116) einschließt, wobei jeder des einen oder der mehreren inkrementellen Anschläge konfiguriert ist, um einen Abstand zu begrenzen, um den der Abschnitt des Arms (110) bewegbar ist, um den Stößel (134) zusammenzudrücken,
wobei der Arm (110) eine Lasche (112) einschließt, die konfiguriert ist, um den Stößel (134) zu berühren und den Stößel niederzudrücken, wenn der Abschnitt des Arms (110) in Richtung der zusammengezogenen Position bewegt wird,
wobei das elastische Element (144) eine Feder einschließt, die konfiguriert ist, um eine erste Kraft auf den Arm (110) auszuüben, und
wobei der Dämpfer (142) einen Kolben einschließt, der konfiguriert ist, um eine zweite Kraft auf den Arm (110) auszuüben, die der ersten Kraft entgegengesetzt ist.

2. Vorrichtung nach Anspruch 1, wobei die Lasche (112) zwischen einer ersten Position, in der die Lasche mit dem Stößel (134) ausgerichtet ist, und einer zweiten Position drehbar ist, in der die Lasche nicht mit dem Stößel ausgerichtet ist.

3. Vorrichtung nach Anspruch 1, wobei der Arm eine oder mehrere Verriegelungslaschen (114) einschließt, die konfiguriert sind, um den Abschnitt des Arms (110) in einer Position lösbar zu verriegeln, die einem des einen oder mehreren inkrementellen Anschläge (116) entspricht, um eine weitere Bewegung des Abschnitts des Arms in Richtung der zusammengezogenen Position zu verhindern.

4. Vorrichtung nach Anspruch 1, wobei die Vorrichtung konfiguriert ist, um eine Druckkraft auf die Feder (144) auszuüben, wenn sich der Abschnitt des Arms (110) in der ausgefahrenen Position befindet.

5. Vorrichtung nach Anspruch 1, wobei der eine oder die mehreren inkrementellen Anschläge (116) so beabstandet sind, dass jeder inkrementelle Anschlag ein inkrementelles Volumen darstellt, das von der Spritze abgegeben wird.

6. Vorrichtung nach Anspruch 1, wobei der Arm (110) als eine Hülse konfiguriert ist, die konfiguriert ist, um über eine Komponente (101D) der Basis zu gleiten.

7. Vorrichtung nach Anspruch 6, wobei der Arm (110) ferner Öffnungen einschließt, die konfiguriert sind, um zu ermöglichen, dass der eine oder die mehreren inkrementellen Anschläge (116), die sich an der Komponente (101D) der Basis befinden, durch ein Ende des Arms hindurchtreten können, wenn sich der Arm in Richtung der zusammengezogenen Position bewegt.

8. Vorrichtung nach Anspruch 2, wobei der Arm ferner eine Sperre einschließt, die konfiguriert ist, um eine Drehung der Lasche in die zweite Position zu verhindern.

## Revendications

1. Dispositif (100) destiné à distribuer un volume au cours d'une perfusion intra-osseuse, le dispositif comprenant :
une base (101) configurée pour être couplée à une seringue (130) présentant un corps définissant un réservoir (132) et un piston plongeur (134) couplé de façon coulissante au corps,
un bras (110) couplé à la base (101), dans lequel une partie du bras est mobile entre une position étendue et une position contractée dans laquelle la partie du bras est plus proche de la base que dans la position étendue,
un élément élastique (144) couplé à la base (101) et au bras (110), dans lequel l'élément élastique (144) est configuré pour solliciter la partie du bras (110) vers la position contractée par rapport à la base avec une force suffisante pour enfoncer un piston plongeur d'une seringue couplée à la base, et
un amortisseur (142) configuré pour résister au déplacement de la partie du bras (110) depuis la position étendue vers la position contractée pour commander une vitesse à laquelle l'élément élastique peut déplacer la partie du bras de la position étendue vers la position contractée,
dans lequel la base (101) définit un canal (106) configuré pour empêcher un déplacement de la seringue le long d'une longueur de la base lorsque le piston plongeur est enfoncé, et la base (101) inclut une ou plusieurs butées incrémentielles (116), chacune des une ou plusieurs butées incrémentielles étant configurée pour limiter une distance sur laquelle la partie du bras (110) est actionnable pour comprimer le piston plongeur (134),
dans lequel le bras (110) inclut une languette (112) configurée pour entrer en contact avec le piston plongeur (134) et enfoncer le piston plongeur lorsque la partie du bras (110) est déplacée vers la position contractée,
dans lequel l'élément élastique (144) inclut un ressort configuré pour exercer une première force sur le bras (110), et
dans lequel l'amortisseur (142) inclut un piston configuré pour exercer une deuxième force sur le bras (110) qui s'oppose à la première force.

2. Dispositif selon la revendication 1, dans lequel la languette (112) peut tourner entre une première position dans laquelle la languette est alignée avec le piston plongeur (134) et une deuxième position dans laquelle la languette n'est pas alignée avec le piston plongeur.

3. Dispositif selon la revendication 1, dans lequel le bras inclut une ou plusieurs languettes de verrouillage (114) configurées pour verrouiller de façon détachable la partie du bras (110) dans une position correspondant à l'une des une ou plusieurs butées incrémentielles (116) pour empêcher un déplacement supplémentaire de la partie du bras vers la position contractée.

4. Dispositif selon la revendication 1, dans lequel le dispositif est configuré pour appliquer une force de compression sur le ressort (144) lorsque la partie du bras (110) est dans la position étendue.

5. Dispositif selon la revendication 1, dans lequel les une ou plusieurs butées incrémentielles (116) sont espacées de sorte que chaque butée incrémentielle représente un volume incrémentiel écoulé à partir de la seringue.

6. Dispositif selon la revendication 1, dans lequel le bras (110) est configuré sous forme d'un manchon configuré pour coulisser sur un composant (101D) de la base.

7. Dispositif selon la revendication 6, dans lequel le bras (110) inclut en outre des ouvertures configurées pour permettre aux une ou plusieurs butées incrémentielles (116) situées sur le composant (101D) de la base de passer à travers une extrémité du bras lorsque le bras de déplace vers la position contractée.

8. Dispositif selon la revendication 2, dans lequel le bras inclut en outre un verrou configuré pour empêcher une rotation de la languette vers la deuxième position.
